# EUROPÄISCHE PATENTSCHRIFT

(11) **EP 2 303 758 B1**
(45) Veröffentlichungstag und Bekanntmachung des Hinweises auf die Patenterteilung: **09.04.2014**
(21) Anmeldenummer: 09802336.9
(22) Anmeldetag: 14.07.2009
(51) Int. Cl.: A61B 17/88, B01F 11/00, B01F 15/02, B67B 7/92

(54) **VORRICHTUNG ZUM ÖFFNEN EINES VERSCHLOSSENEN FLUIDBEHÄLTERS**
DEVICE FOR OPENING A CLOSED FLUID CONTAINER
DISPOSITIF POUR OUVRIR UN RÉCIPIENT DE FLUIDE FERMÉ

(30) Priorität: 29.07.2008 CH 11862008; 21.01.2009 CH 89092009
(43) Veröffentlichungstag der Anmeldung: 06.04.2011
(73) Patentinhaber: Medmix Systems AG, 6343 Rotkreuz (CH)
(72) Erfinder: GRETER, Andy, 6340 Baar (CH); BREM, William, 5630 Muri (CH); KELLER, Wilhelm, A., 6402 Merlischachen (CH)
(74) Vertreter: Detken, Andreas
(86) Internationale Anmeldenummer: PCT/CH2009/000251
(87) Internationale Veröffentlichungsnummer: WO 2010/012114

(56) Entgegenhaltungen:
- EP-A- 0 079 983
- DE-A1- 2 921 565
- DE-A1- 19 532 015
- DE-A1- 19 841 722

## Beschreibung

### TECHNISCHES GEBIET

Die vorliegende Erfindung betrifft eine Öffnungsvorrichtung zum Öffnen eines verschlossenen Fluidbehälters gemäß dem Oberbegriff des Anspruchs 1 and wie aus der DE 195 32 015 bekannt, und ein System zum Mischen wenigstens zweier Komponenten. Insbesondere betrifft die vorliegende Erfindung eine Öffnungsvorrichtung zum Öffnen einer Ampulle mit einem abtrennbaren Ampullenteil und ein System zum Mischen wenigstens zweier Komponenten, wovon wenigstens eine der Komponenten in einer solchen Ampulle aufbewahrt wird.

### STAND DER TECHNIK

In verschiedenen Bereichen der Medizin und der Technik ist es erforderlich, Stoffe zur Behandlung oder Verarbeitung erst kurz vor ihrer Verwendung zu mischen, da das fertige Gemisch zum Beispiel nicht stabil ist oder zum Beispiel seine Handhabung in getrennten Komponenten bis zur Verwendung vorteilhaft ist. Im medizinischen Bereich ist dies zum Beispiel bei der Verarbeitung von Knochenzement wie etwa bei Knochenrekonstruktionen oder beim Einbau von Implantaten wie Hüftgelenken oder für Bandscheibenrekonstruktionen bekannt. Ferner werden derartige Stoffe zum Beispiel bei der Gewebe- oder Wundbehandlung oder zum Stoppen von Blutungen verwendet. Beispielsweise werden Klebstoffe für Wunden erst kurz vor deren Verwendung aus zwei oder mehr Komponenten gemischt. Weitere Anwendungen, die eine solche Mischtechnik erfordern, ergeben sich in der Pharmazie, da manche Medikamente erst kurz vor deren Anwendung gemischt werden können.

Bei der Herstellung von Knochenzement (PMMA) wird zum Beispiel ein fluides Monomer, im Allgemeinen MMA (monomeres Methylmethacrylat), mit einer pulverförmigen Komponente vermischt. Das Fluid ist im Allgemeinen in einer länglichen Glasampulle vorgesehen, die an einem Ende einen Kopfbereich aufweist, der von der Ampulle durch einen leichten Schlag oder eine Biegekraft abgetrennt werden kann. Meist ist an diesem abtrennbaren Teil eine Sollbruchstelle vorgesehen. Die pulverförmige Komponente ist in einem weiteren Behälter vorgesehen, der zeitweise gleichzeitig auch als Mischbehälter dienen kann. Erst kurz vor der Anwendung des Knochenzements wird die Ampulle mit dem fluiden Monomer aufgebrochen und das Fluid der trockenen Komponente hinzugefügt und mit dieser vermischt. Beim Aufbrechen der Ampulle besteht ein Verletzungsrisiko an den Bruchkanten und es besteht die Gefahr, dass Splittermaterial in das Fluid gelangen. Im Weiteren ist das Personal den aggressiven Dämpfen ausgesetzt.

Aus dem Stand der Technik sind mehrere Einrichtungen bekannt, die das Aufbrechen eines Fluids enthaltenden Behälters und das Vermischen des Fluids mit einer weiteren Komponente erleichtern. Aus der US 6,296,149 ist beispielsweise eine Vorrichtung bekannt, bei welcher der Kopf einer Ampulle durch eine Drehvorrichtung abgetrennt wird. In der Drehvorrichtung sind mehrere Trennelemente angeordnet, die bei einem Drehen gegen den Kopf einer Ampulle schlagen und diesen abtrennen können. Dabei wird die Ampulle in einem Gehäuse gehalten und die Drehvorrichtung relativ zum Gehäuse verdreht. Die Drehachse, um welche die Drehvorrichtung gedreht wird, ist parallel und versetzt zur Ampullenachse vorgesehen. Die Drehvorrichtung muss daher baulich weit über das Ampullengehäuse hinausragen, so dass die Gesamtheit aus Ampullengehäuse und Drehvorrichtung sehr ausladend gestaltet ist. Zum Verdrehen der Drehvorrichtung muss das Ampullengehäuse mit einer Hand und die Drehvorrichtung mit einer anderen Hand gefasst werden. Zum Abtrennen des Ampullenkopfes erfolgt eine Krafteinwirkung ausschliesslich über die relative Drehbewegung dieser beiden Teile zueinander und muss gegen die Reibungskräfte der Drehvorrichtung und die Gegenkraft des Ampullenkopfes wirken. Zum Abtrennen des Ampullenkopfes ist daher ein grosser Kraftaufwand notwendig.

In der EP 0 972 499 ist eine Einrichtung zum Aufbrechen einer Ampulle vorgesehen, bei der ein Basiselement zur Aufnahme der Ampulle und ein Kappenelement, das auf das Basiselement aufgeschraubt werden kann, vorgesehen sind. In der Kappe ist eine Bruchstruktur vorgesehen, welche beim Zusammenschrauben des Basiselements mit der Kappe einen Boden der Ampulle aufbricht. Bei dieser Vorrichtung zum Aufbrechen der Ampulle werden zwei Elemente entlang einer einzigen Drehachse zueinander verdreht. Auch bei dieser Vorrichtung wird die Ampulle durch eine reine Rotationskraft beim Zusammendrehen der Bauelemente aufgebrochen. Ferner besteht die Gefahr, das der aufgebrochene Teil der Ampulle in das Innere der Ampulle fällt und somit das Fluid durch Splitter und dergleichen verunreinigt wird.

WO 97/07748 (fami der DE 195 32 015) offenbart eine Vorrichtung zum Mischen und Austragen eines Produkts aus zwei Komponenten. Eine der beiden Komponenten ist pulverförmig und befindet sich zusammen mit einem Mischelement und Austragelementen in einer Kammer. Die andere Komponente ist flüssig und ist in einer Ampulle gelagert. Die Ampulle ist in einem Gehäuse aufgenommen, das gegenüber der Kammer um eine quer zur Ampullenlängsachse verlaufende Schwenkachse verschwenkbar ist. Dabei wird der Ampullenkopf in einem Lagerstutzen ortsfest zur Kammer gehalten und bricht durch die Schwenkbewegung vom Ampullenkörper ab. Die dadurch aus der Ampulle austretende zweite Komponente wird in Richtung der Schwenkachse seitlich aus dem Gehäuse in die Kammer mit der ersten Komponente übergeleitet. Hierzu ist zwischen Gehäuse und Kammer eine verhältnismässig komplizierte Anordnung von Kanälen vorhanden.

Die Handhabung dieser Vorrichtung ist relativ umständlich, und eine Entleerung nicht nur des Ampullenkörpers, sonder auch des Ampullenkopfes ist nur gewährleistet, wenn die gesamte Vorrichtung in einer geeigneten Orientierung gehalten wird, in der der Ampullenkopf nach oben weist. Auch die Anordnung der Kanäle zur Überleitung der zweiten Komponente in die Kammer mit der ersten Komponente ist sehr kompliziert und dadurch teuer in der Herstellung und störanfällig. Es ist nicht vorgesehen und ohne konstruktive Änderungen auch nicht durchführbar, dass die Einrichtung zum Brechen der Ampulle getrennt von der Misch- und Austragseinrichtung ausgebildet ist, sondern diese Einrichtungen bilden eine untrennbare Einheit.

### DARSTELLUNG DER ERFINDUNG

Es ist eine Aufgabe der vorliegenden Erfindung, eine Öffnungsvorrichtung zum Öffnen eines verschlossenen Fluidbehälters zu schaffen, die grundsätzlich unabhängig von einer Mischund Austragvorrichtung ausgebildet werden kann, die in einfacher Weise bedient werden kann, und die den sicheren Umgang mit den verwendeten Mischkomponenten ermöglicht. Ausserdem ist erwünscht, dass wenig Kraftaufwand zum Öffnen des Fluidbehälters erforderlich ist, dass die Vorrichtung ein platzsparendes Design aufweist und insbesondere eine vorteilhafte Ausnutzung der angewendeten Kräfte vorsieht. Ferner ist es eine Aufgabe der vorliegenden Erfindung, ein System zum Mischen wenigstens zweier Komponenten zu schaffen, bei welchem die Komponenten mit nur wenigen Handgriffen zusammengeführt werden können und das sicher und hygienisch in der Handhabung ist.

Diese Aufgaben werden gemäss der vorliegenden Erfindung durch eine Öffnungsvorrichtung zum Öffnen eines verschlossenen Fluidbehälters nach Anspruch 1 und ein System zum Mischen wenigstens zweier Komponenten nach Anspruch 12 gelöst.

Eine Öffnungsvorrichtung zum Öffnen eines verschlossenen Fluidbehälters gemäss der Erfindung umfasst:
ein Gehäuse mit einer ersten Kammer zur Aufnahme des Fluidbehälters und mit einer sich an die erste Kammer anschliessenden, zur ersten Kammer hin offenen zweiten Kammer mit einem Auslass, wobei die zweite Kammer dazu ausgebildet ist, einen abtrennbaren Teil des Fluidbehälters aufzunehmen; sowie
eine um eine Drehachse drehbar in der zweiten Kammer angeordnete Drehtrommel mit wenigstens einem Trennelement, das in einer Ausgangsposition versetzt zum abtrennbaren Behälterteil angeordnet ist und durch eine Drehung der Drehtrommel um die Drehachse in eine Trennposition bringbar ist, in der es den abtrennbaren Behälterteil vom Fluidbehälter trennt,
wobei die erste und die zweite Kammer eine Verbindungsachse definieren und die Drehachse der Drehtrommel gewinkelt zu dieser Verbindungsachse verläuft,
wobei die zweite Kammer trommelartig ausgestaltet ist und eine sich um die Drehachse der Drehtrommel erstreckende Mantelwand aufweist, und
wobei der Auslass der zweiten Kammer in der Mantelwand der zweiten Kammer angeordnet ist.

Ein abtrennbarer Teil des Fluidbehälters ragt also von der ersten Kammer in die zweite Kammer hinein. Als Kammer sind auch nur verschiedene Bereiche des Gehäuses zu verstehen, wobei der abtrennbare Teil des Fluidbehälters in einem Bereich und der Rest des Behälters in einem anderen Bereich zu liegen kommt. Ferner umfasst die Öffnungsvorrichtung wenigstens ein drehbar oder schwenkbar in der zweiten Kammer gelagertes Trennelement, das in einer Ausgangsposition versetzt zum abtrennbaren Behälterteil angeordnet ist und in eine Trennposition drehbar oder schwenkbar ist, in der es den abtrennbaren Behälterteil vom Fluidbehälter trennt. Die Drehachse der Drehtrommel ist gewinkelt zu einer Verbindungsachse zwischen erster und zweiter Kammer des Gehäuses, bzw. zur Achse des Fluidbehälters angeordnet. Dadurch wird eine sehr einfache Bedienung möglich. Der Auslass ist in der Mantelwand der zweiten Kammer angeordnet. Dadurch ist es sehr einfach möglich, das Fluid aus der zweiten Kammer zu entnehmen. Ausserdem wird es so möglich, am Auslass ein Arlschlussstück vorzusehen, über das die Öffnungsvorrichtung auf einfache Weise lösbar mit einem weiteren Teil, z.B. einer Mischund Austragvorrichtung, verbunden werden kann.

Das Gehäuse ist vorzugsweise länglich ausgebildet. Die erste Kammer kann hülsenartig geformt sein. Die zweite Kammer ist trommelartig ausgebildet und schliesst sich an die erste Kammer an, wobei die Kammern derart aneinander gereiht sind, dass sich eine Verbindungsachse in Richtung der Aneinanderreihung ergibt. Die beiden Achsen der beiden Kammern sind gewinkelt, vorzugsweise senkrecht zueinander angeordnet. Der Fluidbehälter kann als herkömmliche Ampulle aus Glas oder Kunststoff vorgesehen sein. Vorzugsweise ist der abtrennbare Behälterteil als eine Art Ampullenkopf mit einer Sollbruchstelle für ein leichtes Abtrennen des Ampullenkopfes versehen. Der Fluidbehälter kann in die erste Kammer des Gehäuses eingeführt werden, bis der abtrennbare Behälterteil in die zweite Kammer hineinragt. Durch Anschläge innerhalb des Gehäuses wird der Fluidbehälter an einem weiteren Vorschieben in das Gehäuse hinein gehindert. Das Gehäuse kann an der Einführstelle für den Fluidbehälter mit einem Verschluss derart verschlossen werden, dass der Fluidbehälter fest in der ersten Kammer gehalten wird. Hierfür kann der Verschluss zum Beispiel ein flexibles Andrückelement, wie etwa einem Gummi, aufweisen, der auf den hinteren Teil der Ampulle drückt. Das Gehäuse kann auch zur Aufbewahrung oder zum Transport des Fluidbehälters verwendet werden.

In der zweiten Gehäusekammer ist das wenigstens eine Trennelement in einer Ausgangsposition versetzt zum abtrennbaren Teil des Fluidbehälters angeordnet. Die versetzte Position des Trennelements kann sich zum Beispiel dadurch ergeben, dass das Trennelement neben oder auch unter dem abtrennbaren Fluidbehälterteil angeordnet ist. Wesentlich ist dabei, dass das Trennelement derart versetzt zur Drehachse des Trennelements vorgesehen ist, dass es durch eine Drehung der Drehtrommel beweglich ist, vorzugsweise auf einer Kreisbahn. Da die Drehachse der Drehtrommel gewinkelt zur Achse des Fluidbehälters, bzw. zur Verbindungsachse zwischen erster und zweiter Gehäusekammer verläuft, wird bei einer Drehung der Drehtrommel das Trennelement auf den abtrennbaren Teil des Fluidbehälters zu bewegt. Sobald das Trennelement auf den abtrennbaren Behälterteil trifft, wirken Kräfte auf den abtrennbaren Teil, die an diesem seitlich angreifen und dadurch den abtrennbaren Teil des Fluidbehälters von dem in der erste Kammer festgehaltenen Fluidbehälterteil abtrennen. Die Druckkraft oder Scherkraft, die von der Seite auf den abtrennbaren Teil drückt, ist ausreichend, um den Teil abzutrennen, ggf. an einer Sollbruchstelle. Grundsätzlich ist es jedoch auch möglich, dass das wenigstens eine Trennelement eine Trennkante oder sogar eine Schneidkante aufweist, die bei einer Drehung auf den abtrennbaren Teil treffen und diesen brechen, bzw. abtrennen. Ferner ist es denkbar, dass das Trennelement über die Trennposition hinaus bewegt werden kann. Vorzugsweise wird jedoch eine Führung vorgesehen, welche die Drehbewegung zwischen den beiden Positionen begrenzt.

Als Achse des Fluidbehälters wird die Achse bezeichnet, entlang derer sich der Hauptteil des Fluidbehälters und der sich daran anschliessende abtrennbare Teil des Fluidbehälters anordnen. Im Falle einer hülsenartigen ersten Gehäusekammer, in welcher eine Ampulle eingesetzt wird, entspricht diese Achse der Achse der Gehäusekammer, bzw. der Achse der Ampulle.

Die Drehachse der Drehtrommel ist gewinkelt zur Verbindungsachse zwischen erster und zweiter Kammer, bzw. zur Achse des Fluidbehälters angeordnet. Vorzugsweise ist der Winkel zwischen den Achsen derart ausgebildet, dass die Drehtrommel seitlich des Gehäuses erfasst und manuell gedreht werden kann. Besonders bevorzugt ist die Drehachse der Drehtrommel im Wesentlichen senkrecht zur Achse des Fluidbehälters vorgesehen.

Die erfindungsgemässe Öffnungsvorrichtung zum Öffnen des Fluidbehälters kann in einer schmale Bauform ausgebildet werden, denn ein Verdrehen der Drehtrommel kann im Wesentlichen innerhalb der Ausmasse des Gehäuses erfolgen. Es sind keine ausladenden Zusatzelemente am Gehäuse notwendig. Das Trennelement ist in platzsparender Weise nahe dem abzutrennenden Teil des Fluidbehälters vorgesehen. Durch die gewinkelte Anordnung der Achsen kann eine Hebelwirkung ausgenutzt werden. Diese entsteht durch die Relativbewegung zwischen der Drehtrommel und dem länglichen Gehäuse. Sofern das Gehäuse mit einer Hand in einem Abstand zur Drehtrommel gehalten und mit der anderen Hand die Drehtrommel gedreht wird, wirkt der Abstand als Hebel und erleichtert die Drehbewegung.

Vorzugsweise setzt das Trennelement oder setzen die Trennelemente an dem abtrennbaren Behälterteil an wenigstens einer Seite des Behälterteils an, besonders bevorzugt an zwei sich gegenüberliegenden Seiten des Behälterteils. Dabei kann der abtrennbare Behälterteil zum Beispiel in eine Öffnung, eine Einbuchtung oder eine Aussparung eines einzelnen Trennelements eingreifen oder zwischen zwei oder mehreren getrennten Trennelementen hindurchragen. Bei einem Verdrehen des Trennelements oder der Trennelemente greift die Scherkraft auf mindestens einer Seite des abtrennbaren Behälterteils an, vorzugsweise auf zwei Seiten, so dass das Abtrennen vereinfacht wird. Besonders bevorzugt ist das Trennelement derart ausgebildet, dass es das abtrennbare Behälterteil nach dem Abtrennen festhält.

Die Drehtrommel kann beinahe formschlüssig in die trommel- oder hülsenartige zweite Behälterkammer eingesetzt sein, so dass sie im Wesentlichen an der Innenwand der Gehäusehülse anliegt. Die Trennelemente können zum Beispiel als vom Boden der Drehtrommel abstehende Stifte oder Vorsprünge vorgesehen sein, wobei sie neben dem Mittelpunkt der Trommel angeordnet sind. Die Drehtrommel kann einen Griff aufweisen, der aus der zweiten Behälterkammer hervorragt und zum Drehen der Drehtrommel in der Kammer vorgesehen ist. Vorzugsweise ist der Griff länglich als eine Art Griffplatte vorgesehen, welche von der Drehtrommel absteht, um ein einfaches Drehen zu ermöglichen. Der Griff kann dabei bezüglich der Drehachse asymmetrisch ausgebildet sein und z.B. an einer Seite über die Umfangswand der Drehtrommel oder die Mantelwand der zweiten Kammer überstehen, um die Drehstellung der Drehtrommel einfacher erkennbar zu machen und die Hebelwirkung zwischen Gehäuse und Drehtrommel zu vergrössern. Grundsätzlich sind aber auch andere Ausgestaltungen des Griffs möglich, solange ein Drehen der Drehtrommel ermöglicht wird.

Die Drehtrommel z. B. wird durch eine Schnappverbindung in die Gehäusekammer eingesetzt und kann vorzugsweise nicht aus dieser entnommen werden. Somit wird ein Verletzungsrisiko durch abgetrennte Behälterteile ausgeschlossen. Es ist jedoch auch denkbar, dass die Drehtrommel aus der zweiten Behälterkammer herausnehmbar ist, so dass ein abgetrenntes Fluidbehälterteil in einfacher Weise entsorgt werden kann.

Die Drehtrommel weist eine erste Öffnung auf, durch welche das abtrennbare Fluidbehälterteil in das Innere der Trommel hineinragt und welche länglich entlang des Umfangs der Drehtrommel vorgesehen ist. Weiter kann die Drehtrommel in ihrer Umfangswand einen Durchgang aufweisen, der in einer Ausgangsposition versetzt zum Auslass der zweiten Behälterkammer angeordnet ist, so dass der Auslass der zweiten Kammer durch die Umfangswand der Drehtrommel verschlossen ist. Wird die Drehtrommel in die Trennposition der Trennelemente gedreht, kommt der Durchgang der Drehtrommel gegenüber dem Auslass der zweiten Behälterkammer zu liegen. Somit entsteht eine Fluidverbindung zwischen dem Inneren der Drehtrommel und dem Auslass aus der zweiten Behälterkammer, so dass das Fluid aus der Öffnungsvorrichtung austreten kann. Die Drehtrommel übernimmt somit die Funktion eines Ventils, welches den Auslass aus dem Gehäuse für das Fluid öffnet oder schliesst.

In einer bevorzugten Ausführungsform weist der Durchgang der Drehtrommel einen Filter auf. Der Filter kann insbesondere Splitter- oder Bruchteile, die beim Abtrennen des Fluidbehälterteils entstanden sind, zurückhalten. Der Filter kann direkt in der Drehtrommel integriert sein oder als separates Bauteil an der Drehtrommel angebracht werden.

In einer weiteren Ausführungsform der vorliegenden Erfindung ist zwischen der zweiten Behälterkammer und der Drehtrommel eine Führung vorgesehen, die zwei Drehanschläge aufweist, einen für eine erste Drehrichtung und einen zweiten für eine zweite Drehrichtung. Wird die Drehtrommel von der Ausgangsposition in einer ersten Drehrichtung in die Trennposition gedreht, wird sie an dem ersten Drehanschlag der Führung gestoppt. In dieser Stoppposition, bzw. Trennposition, ist der abtrennbare Teil des Fluidbehälters von dem Fluidbehälter abgetrennt und der Durchgang der Drehtrommel liegt dem Auslass des Gehäuses gegenüber. In dieser definierten Position kann das Fluid aus dem Fluidbehälter und aus dem Gehäuse der Vorrichtung austreten. Wird die Drehtrommel in der entgegengesetzten Drehrichtung von der Trennposition in die Ausgangsposition gedreht wird die Drehung durch den zweiten Drehanschlag der Führung gestoppt. In dieser zweiten Stoppposition liegt die Umfangswand der Drehtrommel dem Auslass der zweiten Gehäusekammer gegenüber und ein Austreten des Fluids aus dem Gehäuse ist nicht möglich. Das Ventil ist in dieser Position geschlossen.

Die Drehtrommel kann also gleichzeitig zwei Funktionen übernehmen. Sie dient zum einen zum Öffnen des Fluidbehälters, in dem der abtrennbare Teil des Fluidbehälters durch Drehen der Drehtrommel abgetrennt wird und zum anderen zur Regulierung des Ausflusses des Fluids aus dem Gehäuse der Vorrichtung.

Bei einer weiteren Ausführungsform kann das Gehäuse einen Vakuumanschluss aufweisen, um in dem Gehäuse einen Unterdruck erzeugen zu können. Vorteilhafterweise wird der Vakuumanschluss durch die Ventilfunktion der Drehtrommel geregelt. Eine solche Öffnungsvorrichtung mit einem Vakuumanschluss kann besonders vorteilhaft bei einem System zum Mischen wenigstens zweier Komponenten verwendet werden, wie nachfolgend genauer ausgeführt wird.

Weiter kann die Öffnungsvorrichtung eine Anzeige aufweisen, welche die Trennposition des Trennelements anzeigt. Eine solche Anzeige kann zum Beispiel durch eine Farb- oder Strichmarkierung auf der Drehtrommel vorgesehen sein, die relativ zu einer Markierung am Gehäuse entsprechend einer Trenn- oder Ausgangsposition verdreht ist. Die Markierung kann aber auch durch den Griff an der Drehtrommel vorgesehen sein, der in einer Ausgangsstellung quer zur Längsachse der ersten Gehäusekammer steht und somit ein geschlossenes Ventil anzeigt und nach einem Verdrehen in Längsrichtung zur Achse der ersten Gehäusekammer steht und somit ein geöffnetes Ventil und einen abgetrennten Fluidbehälterteil anzeigt. Selbstverständlich sind weitere Ausgestaltungen einer Anzeige möglich.

Weiter ist es bei einer vorteilhaften Ausgestaltung der Erfindung vorgesehen, dass an dem Auslass der zweiten Kammer des Fluidbehälters ein Anschlussstück vorgesehen ist, mit welchem das Gehäuse an ein weiteres Bauelement, wie zum Beispiel einen weiteren Behälter angeschlossen werden kann. Ein solches Anschlussstück kann zum Beispiel als Teil einer Gewindeverbindung, einer Luer Lock Verbindung oder einer Bajonettverbindung vorgesehen sein.

Nach einem weiteren Aspekt der vorliegenden Erfindung ist ein System zum Mischen wenigstens zweier Komponenten vorgesehen, das wenigstens eine Öffnungsvorrichtung wie oben dargestellt und einen Mischbehälter umfasst, an den die wenigstens eine Öffnungsvorrichtung unter Herstellung einer Fluidverbindung fest oder abnehmbar angeschlossen ist. Dabei entsteht eine Fluidverbindung zwischen dem Gehäuse der Öffnungsvorrichtung und dem Innenraum des Mischbehälters. Hierfür kann der Mischbehälter zum Beispiel ein Anschlussstück aufweisen, das mit dem Anschlussstück am Auslass der zweiten Gehäusekammer der Öffnungsvorrichtung zusammenwirkt. Die Öffnungsvorrichtung kann demnach zum Beispiel auf den Mischbehälter aufgeschraubt oder mittels einer Bajonettverbindung aufgesetzt werden. Es kann zum Beispiel auch ein Schliessmechanismus, wie etwa eine Membran, vorgesehen sein, welcher die Öffnung des Mischbehälters vor dem Aufsetzen der Öffnungsvorrichtung verschliesst und beim Aufsetzen geöffnet wird.

In einer Ausführungsform ist das System für eine erste Komponente in Form eines Fluids in einem Fluidbehälter und eine zweite Komponente in Form eines Feststoffs vorgesehen, wobei der Feststoff vorzugsweise bereits in dem Mischbehälter untergebracht ist. Der Feststoff kann natürlich ebenfalls in einem zusätzlichen Behälter vorgesehen sein und erst vor dem Mischen in den Mischbehälter gefüllt werden. Grundsätzlich ist es möglich, weitere Öffnungsvorrichtungen mit weiteren Komponenten an weitere Anschlussstücke an den Mischbehälter anzuschliessen. Ferner können auch herkömmliche Fluidbehälter oder andere Behälter zusätzlich an den Mischbehälter angeschlossen werden.

Das System ist zum Mischen jeglicher Art von Mehrkomponentenmischungen geeignet. Besonders vorteilhaft kann es für Mehrkomponentenanwendungen mit aggressiven oder leicht flüchtigen Stoffen, die in einer Glasampulle aufbewahrt sind, verwendet werden. Als ein Anwendungsbeispiel sei das Mischen von Knochenzement genannt, bei welchem in der erfindungsgemässen Öffnungsvorrichtung ein Monomer, wie zum Beispiel MMA, vorgesehen ist und in dem Mischbehälter bereits der pulverartige Zementstoff aufbewahrt ist. Mit einem System nach der vorliegenden Erfindung können z. B. auch Gele abgemischt werden.

In einer Ausgestaltung des Mischsystems kann der Mischbehälter ein im Mischbehälter beweglich gelagertes Mischpaddel oder ein Rührpaddel aufweisen, um die Komponenten innerhalb des Mischbehälters zu durchmischen. Je nach Viskosität der Mischung kann das Paddel hierfür entsprechend ausgebildet werden. Der Mischvorgang kann vor oder nach dem Abnehmen der Öffnungsvorrichtung von dem Mischbehälter erfolgen. Das Mischoder Rührpaddel kann zum Beispiel an einer Kolbenstange vorgesehen sein, die an einem Ende aus dem Mischbehälter ragt und am anderen Ende eine Mischvorrichtung zum Beispiel in Form eines Paddels aufweist. Zur Verabreichung wird vorzugsweise die Kolbenstange von dem Paddel abgenommen, bzw. abgebrochen und es wird ein Austragungsmechanismus, z. B. in Form einer Druckpistole, an die Mischkammer angeschlossen. Anstelle der Öffnungsvorrichtung kann an das Anschlussstück des Mischbehälters eine Düse, ein Schnorchel oder eine Kanüle angeschlossen werden, die als Abgabeeinrichtung für das Mischerzeugnis dienen. Durch diese Abgabeeinrichtung kann das Mischerzeugnis aus dem Mischbehälter mittels des Austragungsmechanismus abgegeben werden.

Es ist auch möglich, dass zur Abgabe der Mischung an dem Paddel ein zusätzliches Flächenelement vorgesehen wird, welches dann als eine Art Stopfen innerhalb des Mischbehälters wirkt und bei Vorschub die Abgabe der Mischung bewirkt. Ein solches Flächenelement kann zum Beispiel an dem Paddel angeordnet werden, wenn dieses mit der Kolbenstange gegen das Flächenelement gedrückt wird, welches sich bereits im Inneren des Mischbehälters befindet, und anschliessend die Kolbenstange abgebrochen wird.

In einer weiteren Ausgestaltung eines erfindungsgemässen Mischsystems ist eine Einrichtung zur Erzeugung eines Unterdrucks im System vorgesehen. Beispielsweise kann die Öffnungsvorrichtung einen Vakuumanschluss aufweisen, durch den im Innenraum des Systems ein Unterdruck erzeugt werden kann. Der Vakuumanschluss kann ein Rückschlagventil aufweisen, um den erzeugten Unterdruck im System nach dem Abnehmen eines Vakuumerzeugers aufrecht erhalten zu können. Mit dem Vakuumanschluss kann das Gehäuse der Öffnungsvorrichtung und somit das Innere der Drehtrommel und der Mischkammer vor dem Abtrennen des Ampullenkopfes mit Unterdruck versehen werden. Beim Öffnen der Ampulle wird somit das Fluid aus der Ampulle gesaugt, da in der Ampulle ein relativ höherer Druck herrscht.

Vorzugsweise ist der Vakuumanschluss an der Gehäusekammer mit der Drehtrommel vorgesehen. Die Erzeugung des Unterdrucks wird vorzugsweise durch die Stellung der Drehtrommel gesteuert. Sofern die Umfangswand der Drehtrommel einem Auslass aus dem Gehäuse oder dem Anschluss für die Erzeugung des Unterdrucks gegenüberliegt, übt sie eine geschlossene Ventilfunktion aus. Wird jedoch die Drehtrommel in eine Position gedreht, in der eine ihrer Öffnungen dem Gehäuseauslass oder dem Unterdruckanschluss gegenüberliegen, übt sie die Funktion eines geöffneten Ventils aus. Die Drehtrommel weist somit eine Vakuumdrehposition auf, in der Vakuum an das System angelegt wird. Die Drehtrommel wird von der Ausgangsposition in die Vakuumposition gedreht, in der die einzelnen Volumina des Gehäuses und des Mischbehälters miteinander verbunden sind. In dieser Stellung ist das Ventil geöffnet. Anschliessend wird z. B. mittels einer Pumpe ein Unterdruck im System erzeugt. Das System kann von der Pumpe getrennt werden, wobei der Unterdruck auf Grund des Rückschlagventils erhalten bleibt. Die Drehtrommel wird in die Trennposition gedreht, in der sie den abtrennbaren Behälterteil abtrennt. Durch das Vakuum wird das Entleeren des Behälters, bzw. der Ampulle, unterstützt. Ferner herrscht auch während dem Mischvorgang ein Unterdruck im System, so dass Lufteinschlüsse in der Mischung reduziert oder ganz ausgeschlossen werden können.

Grundsätzlich ist auch denkbar einen Anschluss zur Erzeugung eines Unterdrucks in dem System an dem Mischbehälter vorzusehen. Dies ist beispielsweise vorteilhaft, wenn in dem Mischbehälter keine Mischkomponenten vorliegen und mehrere Behälter mit Mischkomponenten an den Mischbehälter angeschlossen werden.

### KURZE BESCHREIBUNG DER ZEICHNUNGEN

Bevorzugte Ausführungsformen der Erfindung werden anhand der Zeichnung dargestellt, die in keiner Weise einschränkend auszulegen ist. Aus den Figuren der Zeichnung offenbar werdende Merkmale der Erfindung sollen als zur Offenbarung gehörend verstanden werden. In den Zeichnungen stellen dar:
- Figur 1: eine Explosionsansicht einer erfindungsgemässen Öffnungsvorrichtung gemäss einer ersten Ausführungsform,
- Figur 2: eine dreidimensionale Ansicht der Drehtrommel der Öffnungsvorrichtung der Fig. 1,
- Figur 3a: eine Schnittdarstellung der Öffnungsvorrichtung der Fig. 1 in einer Ausgangsposition,
- Figur 3b: eine Darstellung nach Figur 3a in einer Aussenansicht,
- Figur 4a: eine Schnittdarstellung der Öffnungsvorrichtung der Fig. 1 in einer Trennposition,
- Figur 4b: eine Darstellung nach Figur 4a in einer Aussenansicht,
- Figur 5a: eine Schnittdarstellung der Öffnungsvorrichtung der Fig. 1 nach dem Abtrennen eines Fluidbehälterteils,
- Figur 5b: eine Darstellung nach Figur 5a in einer Aussenansicht,
- Figur 6: eine Explosionsdarstellung eines Systems zum Mischen wenigstens zweier Komponenten mit der Öffnungsvorrichtung gemäss der ersten Ausführungsform,
- Figur 7a: eine Schnittdarstellung einer Öffnungsvorrichtung gemäss einer zweiten Ausführungsform, mit einem Vakuumanschluss, in einer geschlossenen Position,
- Figur 7b: eine Darstellung nach Figur 7a in einer Aussenansicht,
- Figur 8a: eine Schnittdarstellung der Öffnungsvorrichtung der Fig. 7a in einer Position zur Erzeugung eines Unterdrucks,
- Figur 8b: eine Darstellung nach Figur 8a in einer Aussenansicht,
- Figur 9a: eine Schnittdarstellung der Öffnungsvorrichtung der Fig. 7a in einer Trennposition,
- Figur 9b: eine Darstellung nach Figur 9a in einer Aussenansicht,
- Figur 10a: eine Schnittdarstellung der Öffnungsvorrichtung der Fig. 7a nach einem Abtrennen eines Fluidbehälterteils mit geschlossenem Ventil,
- Figur 10b: eine Darstellung nach Figur 10a in einer Aussenansicht,
- Figur 11a: eine schematische Schnittdarstellung eines Systems zum Mischen mit einer Öffnungsvorrichtung gemäss einer dritten Ausführungsform, in einer Ausgangsposition; sowie
- Figur 11b: eine schematische Schnittdarstellung eines Teils des Systems der Fig. 11a in einer Trennposition.

### BESCHREIBUNG BEVORZUGTER AUSFÜHRUNGSFORMEN

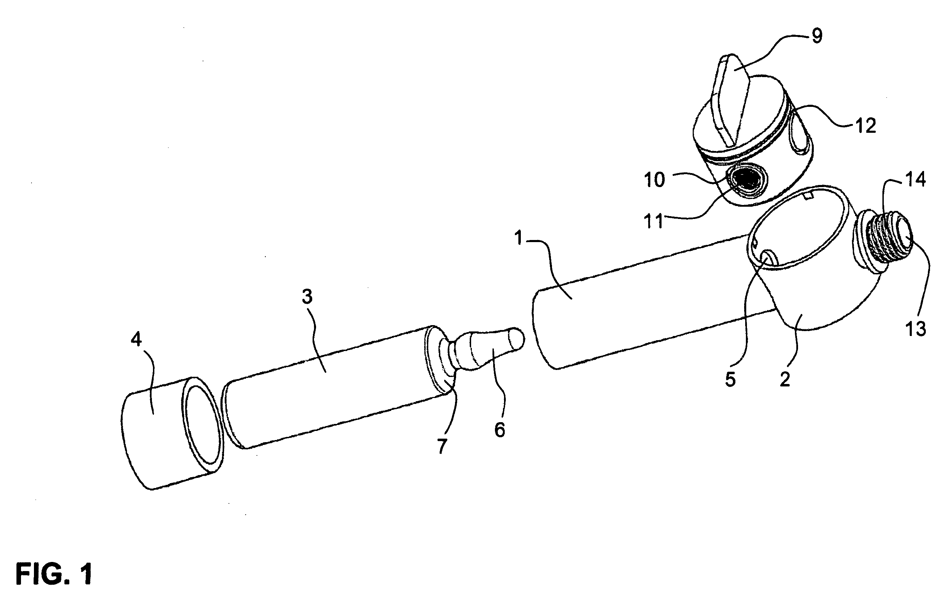

In Figur 1 ist eine erste Ausführungsform einer Öffnungsvorrichtung zum Öffnen eines verschlossenen Fluidbehälters gemäss der vorliegenden Erfindung in einer Explosionsdarstellung der einzelnen Bauelemente gezeigt. Die Öffnungsvorrichtung umfasst ein Gehäuse mit einer ersten Gehäusekammer 1 und einer sich daran anschliessenden zweiten Gehäusekammer 2. Die erste Gehäusekammer 1 ist hülsenartig ausgebildet und zur Aufnahme eines Fluidbehälters in Form einer Glasampulle 3 geeignet. Auf das eine Ende der ersten Kammer 1 ist eine Verschlusskappe 4 aufsetzbar. An das andere Ende der Kammer 1 schliesst sich die zweite Gehäusekammer 2 an, die ebenfalls hülsenartig ausgebildet ist. Die Achsen der ersten Gehäusekammer und der zweiten Gehäusekammer sind in einem 90° Winkel zueinander angeordnet. Im Übergang von der ersten Gehäusekammer zu der zweiten Gehäusekammer ist eine Öffnung 5 vorgesehen. Die Glasampulle 3, welche einen abtrennbaren Kopfteil 6 aufweist, wird an dem einen, offenen Ende der ersten Gehäusekammer 1 in die erste Gehäusekammer 1 eingeführt, bis ihr Kopfteil 6 durch die Öffnung 5 in die zweite Gehäusekammer 2 hindurchragt und sie mit ihrer Schulter 7 gegen die Umfangskante der Öffnung 5 stösst. Anschliessend wird die Verschlusskappe 4 auf die Öffnung der ersten Gehäusekammer 1 aufgesetzt, so dass die Ampulle 3 sicher in dem Gehäuse der Öffnungsvorrichtung untergebracht ist.

In die zweite Gehäusekammer 2 ist eine Drehtrommel 8 einsetzbar. Die Drehtrommel ist ebenfalls hülsenartig ausgebildet und ist passgenau für die zweite Gehäusekammer 2 ausgelegt. Die Drehtrommel 8 weist einen Griff 9 auf, der in Form einer aufgestellten Platte von einer Seitenfläche der Drehtrommel 8 senkrecht abragt. Auf der Umfangsfläche der Drehtrommel 8 ist ein Durchgang 10 vorgesehen, der mit einem Filter 11 versehen ist. Der Durchgang 10 ist am Aussenumfang in der Verlängerungslinie des Griffs 9 angeordnet. Ringförmig um den Aussenumfang der Drehtrommel 8 ist ein Dichtring 12 vorgesehen, der beim Einsetzen der Drehtrommel 8 in die zweite Gehäusekammer 2 den Zwischenraum zwischen dem Aussenumfang der Drehtrommel und dem Innenumfang der zweiten Gehäusekammer abdichtet.

Die Gehäusekammer 2 weist an einer Seite, welche der ersten Gehäusekammer 1 gegenüberliegt, in ihrer Mantelwand (Umfangswand) einen Auslass 13 auf. Dieser Auslass mündet in ein Anschlussstück 14, das auf seinem Aussenumfang ein Gewinde aufweist.

Die Verbindungsachse zwischen der ersten Kammer 1 und der zweiten Kammer 2 des Gehäuses stimmt bei dieser Ausführungsform im Wesentlichen mit der Achse der hülsenartigen Gehäusekammer 1 überein, da sich die Gehäusekammer 2 in dieser Richtung an die erste Kammer anschliesst. Die Drehtrommel 8 ist derart drehbar in die zweite Gehäusekammer 2 einsetzbar, dass die Drehachse der Drehtrommel im Wesentlichen senkrecht auf der Verbindungsachse der Gehäusekammern steht. Es wäre auch denkbar, die ringförmige zweite Gehäusekammer derart an der ersten Gehäusekammer anzubringen, dass die Achse der Drehtrommel zum Beispiel in einem Winkel zwischen 60° - 90° angeordnet ist.

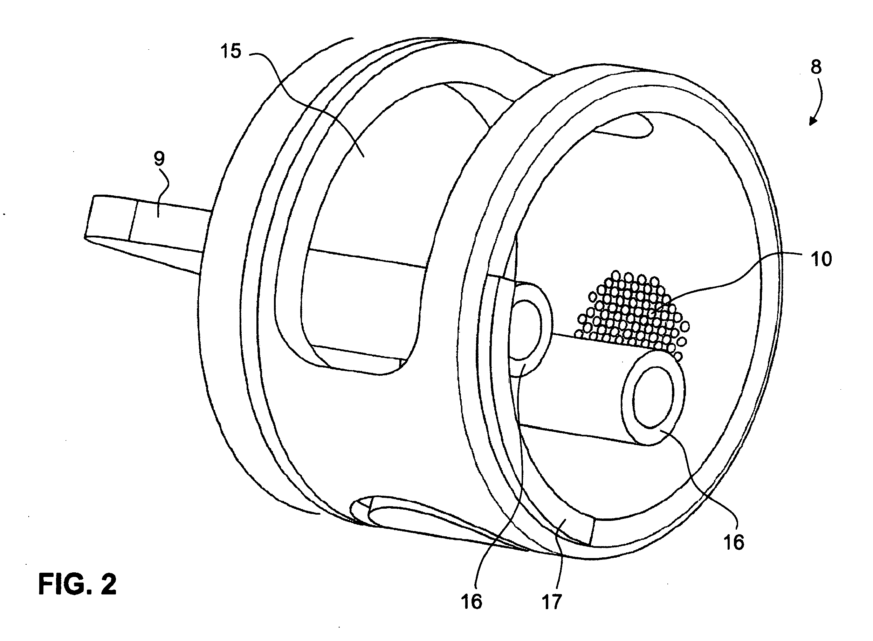

In Figur 2 ist eine dreidimensionale Darstellung der Drehtrommel 8 gezeigt. Auf der linken Seite der Darstellung ist der Griff 9 ersichtlich und auf der rechten Seite der Darstellung ist die Drehtrommel aufgeschnitten gezeigt. In der Umfangswand ist der Durchgang 10 in Form einer Vielzahl von kleinen Öffnungen in der Wand der Drehtrommel gezeigt. Der Filter 11 wird dadurch gebildet, dass der Durchmesser der Löcher klein genug ist, um auszufilternde Bestandteile zurück zu halten. Ferner ist in der Umfangswand der Drehtrommel 8 eine längliche Öffnung, bzw. ein Langloch 15 vorhanden, das sich in einem Sektor von ca. 90° auf dem Umfang der Drehtrommel erstreckt und dessen Öffnung derart gross ist, dass das Kopfteil 6 der Ampulle durch das Langloch hindurch passt und in das Innere der Drehtrommel 8 ragen kann. Von einer inneren Seitenfläche der Drehtrommel 8 ragen zwei Stifte 16 ab und in das Innere der Drehtrommel hinein. Die Stifte 16 bilden gemeinsam das Trennelement. Grundsätzlich kann erfindungsgemäss jedoch auch nur ein derartiger Stift 16 als Trennelement vorgesehen werden. Die Stifte 16 sind jeweils versetzt zum Mittelpunkt der Drehtrommel 8 angeordnet, so dass sie bei einem Verdrehen der Drehtrommel um den Mittelpunkt verdreht oder verschwenkt werden.

Auf der Seite der Drehtrommel, welche der Seite mit dem Griff gegenüberliegt, und die auf einer Bodenfläche der Gehäusekammer 2 zu liegen kommt, ist eine Führungskante 17 angeordnet. Auf der Bodenfläche der Gehäusekammer 2 wiederum ist eine Führungsnut vorgesehen, in welche die Führungskante 17 eingreifen kann. Die Führungsnut ist beispielsweise ringförmig um 180° in die Bodenfläche eingelassen. Die Führungskante 17 wiederum erstreckt sich über einen Bereich von 90° auf der Aussenseite der Drehtrommel. Daraus ergibt sich, dass die Drehtrommel innerhalb der Führungsnut um 90° drehbar ist, bis sie jeweils an den Enden der Führungsnut anschlägt.

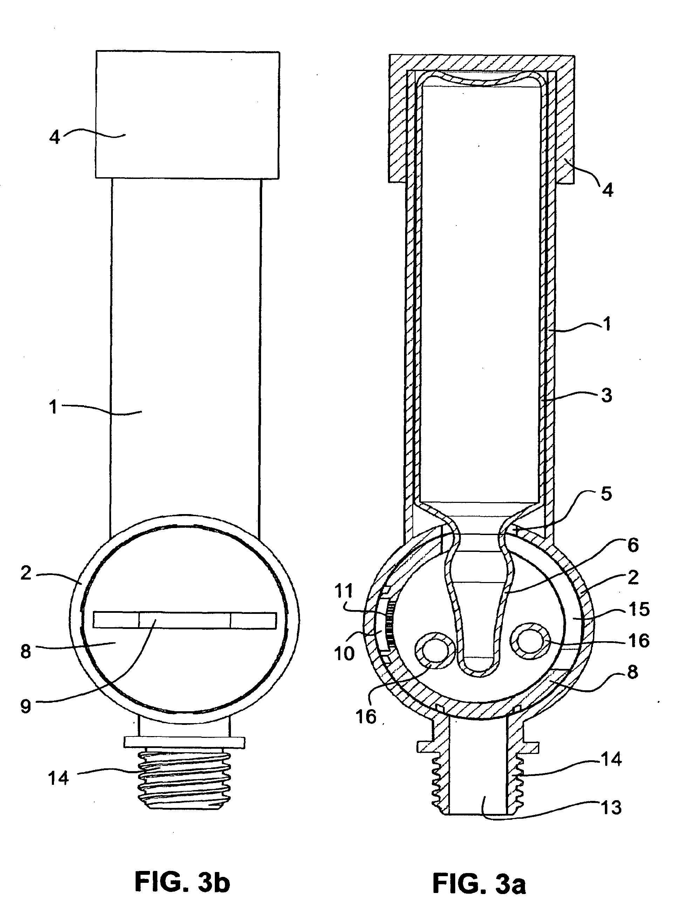

In Figur 3a ist die Öffnungsvorrichtung in einem zusammengesetzten Zustand in einer Schnittdarstellung gezeigt. Die Ampulle 3 ist in die erste Gehäusekammer 1 eingesetzt, und die Kammer ist mit der Verschlusskappe 4 geschlossen. Der Kopfteil 6 der Ampulle ragt durch die Öffnung 5 in die zweite Gehäusekammer 2 und in das Innere der Drehtrommel 8. Der Kopfteil 6 der Ampulle kommt zwischen den beiden Stiften 16 zu liegen. Die Drehtrommel 8 ist in Figur 3a in einer Ausgangsposition gezeigt. Dabei kommt die Umfangsfläche der Drehtrommel 8 gegenüber dem Auslass 13 zu liegen und verschliesst diesen. Der Durchgang 10 der Drehtrommel 8 wiederum kommt gegenüber der Innenumfangsfläche (inneren Mantelfläche) der zweiten Gehäusekammer 2 zu liegen. Das Innere des Gehäuses der Öffnungsvorrichtung ist somit in der Ausgangsposition nach aussen verschlossen.

In Figur 3b ist die Aussenansicht der Schnittdarstellung aus Figur 3a gezeigt. Der Griff 9 der Drehtrommel 8 ist quer zur Längsachse der Ampulle 3 im Inneren des Gehäuses ausgerichtet. Durch diese Querstellung wird angezeigt, dass sich der Durchgang 10 und der Auslass 13 nicht decken und somit das Innere der Drehtrommel 8 nach aussen verschlossen ist. Die Drehtrommel 8 übernimmt somit eine Ventilfunktion für den Durchfluss des Fluids sobald dieses aus der Ampulle austritt, wobei in dieser Position das Ventil geschlossen ist.

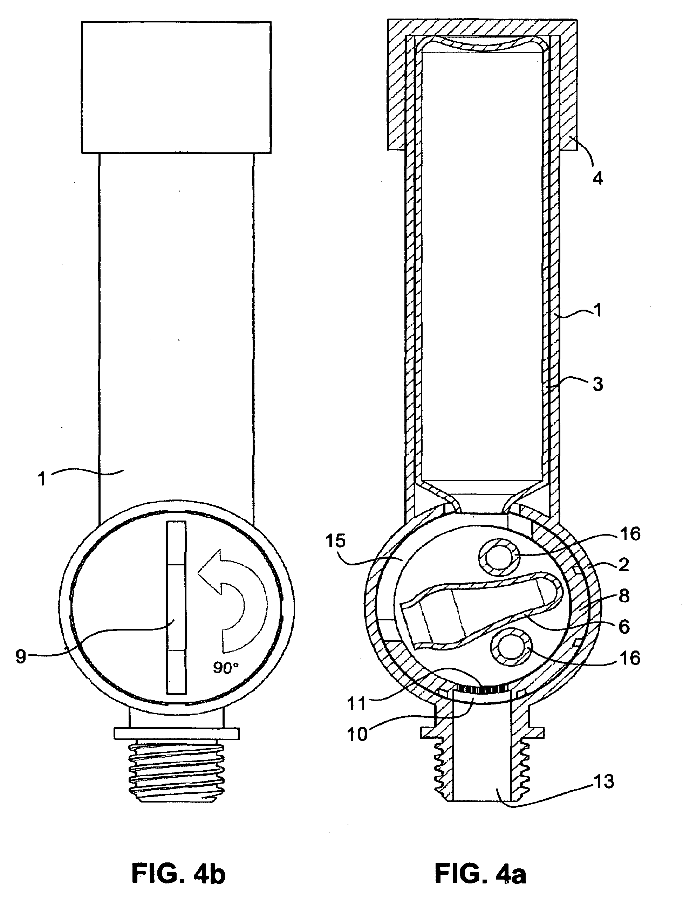

In Figur 4a ist die Öffnungsvorrichtung in einer Trennposition gezeigt, in der das abtrennbare Kopfteil 6 von der Glasampulle 3 abgetrennt ist. Hierfür wurde die Drehtrommel 8 innerhalb der zweiten Gehäusekammer 2 um 90° verdreht. Dabei werden die Stifte 16 mitverdreht und schlagen an der Aussenseite des Kopfteils 6 der Glasampulle 3 an. Es wird eine Kraft auf den Kopfteil 6 übertragen und der Kopfteil bricht von der Ampulle 3 ab. Die beiden Stifte 16 greifen dabei an gegenüberliegende Seiten des Kopfteils 6 an. Die Stifte 16 sind derart dicht zur Aussenfläche des Kopfteils 6 angeordnet, dass der Kopfteil 6 nach dem Abbrechen von der Ampulle 3 von den beiden Stiften 16 gehalten wird.

Bei dem Verdrehen der Drehtrommel 8 wird gleichzeitig der Durchgang 10 mit dem Filter 11 in Deckung mit dem Auslass 13 gebracht. Dadurch entsteht eine Fluidverbindung zwischen dem Innenraum der Drehtrommel 8, der zweiten Gehäusekammer 2 und der Aussenseite des Gehäuses.

Da das Kopfteil 6 der Glasampulle 3 von dieser abgebrochen wurde, kann das Fluid aus dem Inneren der Ampulle 3 durch das Langloch 15 hindurch in das Innere der Drehtrommel 8 fliessen. Von dort kann es durch den Filter 11 und den Durchgang 10 und den Auslass 13 aus dem Gehäuse herausfliessen. Der abgebrochene Kopfteil 6 wird von den Stiften 16 derart gehalten, dass er den Durchgang 10 mit dem Filter 11 nicht blockiert und möglicherweise in dem Kopfteil zurückgebliebenes Fluid kann aus dem Kopfteil abfliessen.

In Figur 4b ist die Aussenansicht der Öffnungsvorrichtung aus Figur 4a gezeigt. Es wird ersichtlich, dass der Griff 9 der Drehtrommel 8 um 90° in die Trennposition gedreht wurde. Der Griff 9 ist daher nun in Längsrichtung der Achse der Glasampulle ausgerichtet und zeigt somit ein geöffnetes Ventil an.

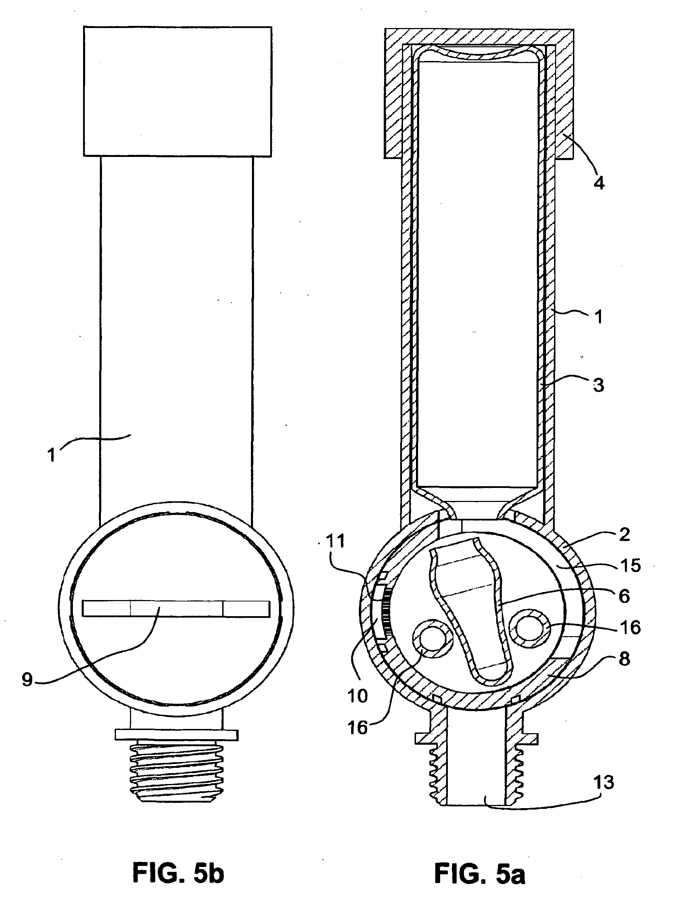

In Figur 5a ist die Öffnungsvorrichtung in einer Verschlussposition gezeigt. In dieser Position wird die Drehtrommel 8 wieder zurückgedreht, bis der Aussenumfang der Drehtrommel dem Auslass 13 gegenüberliegt und der Durchgang 10 mit dem Filter 11 dem Innenumfang der Gehäusekammer 2 gegenüberliegt, so dass das durch die Drehtrommel 8 und die zweite Gehäusekammer 2 gebildete Ventil geschlossen ist. Das Kopfteil 6 wird weiterhin von den Stiften 16 gehalten. Durch die Ventilfunktion kann zum Beispiel die Menge eines Fluids, welches aus dem Gehäuse austreten soll, reguliert werden. Ferner wird verhindert, dass das Mischprodukt in die Drehtrommel gelangt. In dieser Verschlussposition kann die Öffnungsvorrichtung entsorgt werden. Der Inhalt der Ampulle konnte durch die Verwendung der Öffnungsvorrichtung auf sichere Weise abgegeben werden, ohne dass ein Verletzungsrisiko durch das Aufbrechen der Ampulle besteht.

In Figur 5b ist die Aussenansicht der Öffnungsvorrichtung aus Figur 5a gezeigt. Es ist ersichtlich, dass die Drehtrommel 8 wiederum um 90° innerhalb der zweiten Gehäusekammer verdreht wurde. Der Griff 9 ist quer zur Längsachse der Ampulle angeordnet und zeigt ein geschlossenes Ventil an.

Das Verdrehen der Drehtrommel 8 von der Ausgangsposition gemäss Figur 3a in die Trennposition gemäss Figur 4a wird durch die Führungskante 17 und die zugehörige Führungsnut in der Gehäusekammer 2 geführt. Durch diese Führung kann die Drehtrommel 8 innerhalb der Gehäusekammer 2 um jeweils 90° gedreht werden. Je nach den Anforderungen einer Anwendung können die Führungsnut und die Führungskante auch andere Winkelstellungen definieren, wie z. B. 60° oder 120°.

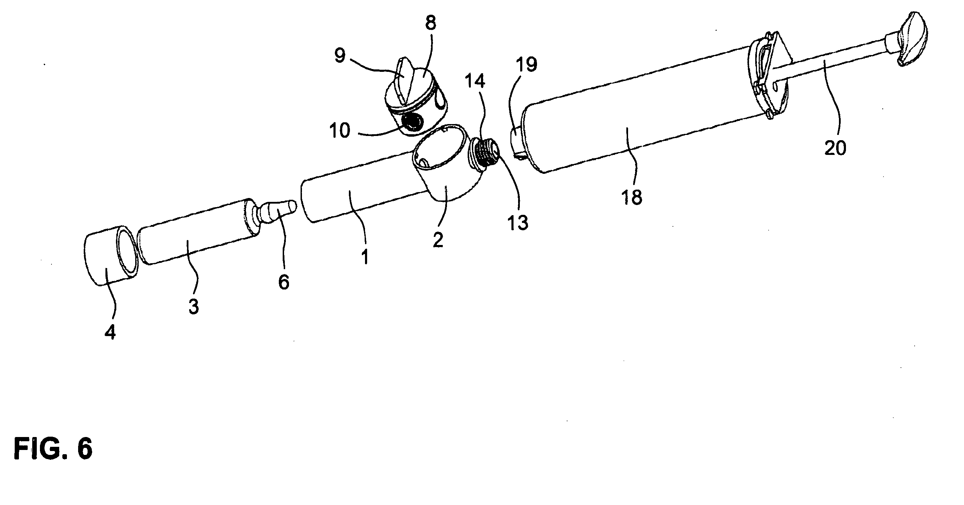

In Figur 6 ist ein System zum Mischen zweier Komponenten gezeigt. Auf der linken Seite der Darstellung sind die Bauteile der Öffnungsvorrichtung abgebildet. Gleiche Bauteile weisen gleiche Bezugszeichen wie in den Figuren 1 bis 5 auf. Auf der rechten Seite der Darstellung ist ein Mischbehälter 18 mit einem Anschlussstück 19 gezeigt, das mit dem Anschlussstück 14 der Öffnungsvorrichtung derart zusammenwirkt, dass eine Fluidverbindung zwischen dem Gehäuse der Öffnungsvorrichtung und dem Inneren des Mischbehälters 18 entsteht. An dem Ende des Mischbehälters 18, das dem Anschlussstück 19 gegenüberliegt, ist ein Mischpaddel 20 mit einer Kolbenstange vorgesehen. Das Mischpaddel 20 ragt in das Innere des Mischbehälters 18 hinein und ist relativ zum Gehäuse des Mischbehälters 18 mittels der Kolbenstange beweglich Um das Gemisch aus dem Mischbehälter auszutragen, wird die Kolbenstange hinter dem Mischpaddel abgebrochen. Mit einem weiteren Applikator wird dann Kraft auf einen Kolben ausgeübt, der die Mischung aus dem Mischbehälter drückt.

In einer Ausführungsform ist im Inneren des Mischbehälters ein Knochenzement in Pulverform vorgesehen. In der Ampulle 3 der Öffnungsvorrichtung ist ein fluides Monomer untergebracht. Zur Herstellung eines Knochenzements wird der Mischbehälter 18 mit dem Anschlussstück 19 auf das Anschlussstück 14 der Öffnungsvorrichtung aufgesetzt. Anschliessend wird die Drehtrommel 8 am Griff 9 mit einer Hand gegriffen, und mit der anderen Hand wird entweder der Mischbehälter 18 oder das Gehäuse 1 erfasst. Mit dieser Handhabung kann die Drehtrommel 8 in einfacher Weise um 90° innerhalb der zweiten Gehäusekammer 2 verdreht werden. Dabei wird das Kopfteil 6 der Ampulle 3 von den Stiften 16 abgebrochen und der Durchgang 10 kommt gegenüber dem Auslass 13 zu liegen. Das Monomer fliesst aus dem Inneren der Glasampulle 3 in die Drehtrommel 8 und von dort durch den Filter 11 des Durchgangs 10 und durch den Auslass 13 über die Fluidverbindung der Anschlussstücke 14 und 19 in das Innere des Mischbehälters 18. Sobald das Fluid in den Mischbehälter 18 geflossen ist, kann die Drehtrommel 8 zurückgedreht werden, so dass das Ventil geschlossen ist. Das in dem Mischbehälter 18 befindliche Monomer und das Zementpulver können nun durch ein Ein- und Ausschieben des Mischpaddels 20 im Inneren des Mischbehälters vermischt werden. Anschliessend kann die Öffnungsvorrichtung von dem Mischbehälter abgenommen werden, und es kann eine Abgabekanüle auf das Anschlussstück 19 des Mischbehälters 18 aufgesetzt werden. Der Knochenzement hat mittlerweile eine Konsistenz angenommen, dass er durch einen Vorschub des Applikators innerhalb des Mischbehälters durch die Abgabekanüle abgegeben werden kann.

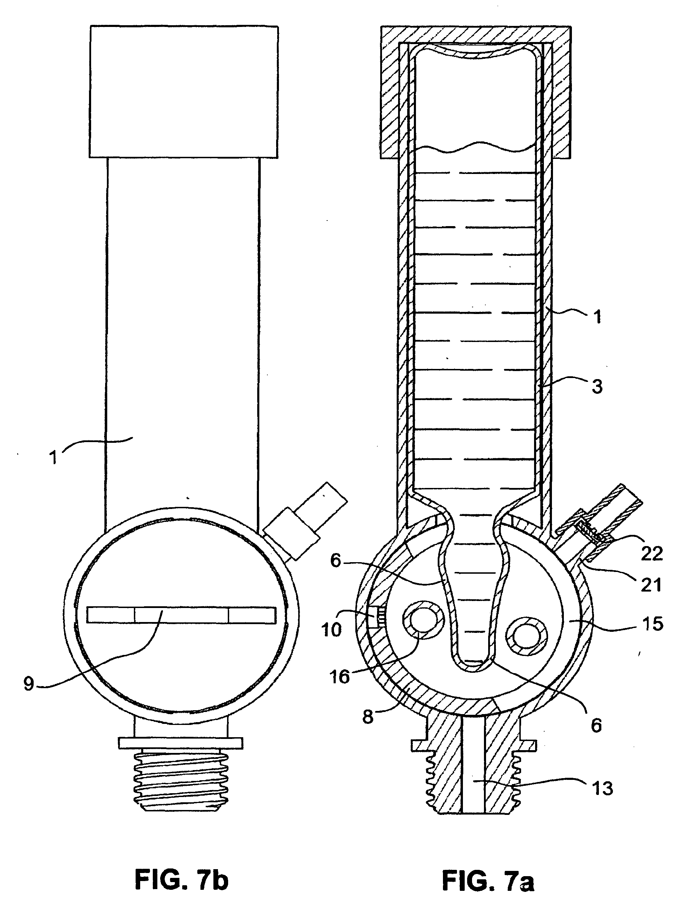

In den Figuren 7 bis 10 wird eine zweite vorteilhafte Ausführungsform einer Öffnungsvorrichtung nach der vorliegenden Erfindung dargestellt. In dieser Ausführungsform ist an der zweiten Gehäusekammer 2 in der Mantelwand bzw. Umfangswand ein weiterer Anschluss 21 vorgesehen, an den eine Einrichtung zur Erzeugung eines Unterdrucks in dem Gehäuse der Öffnungsvorrichtung angeschlossen werden kann. Der Anschluss ist als Kanalstumpf ausgebildet, so dass z. B. eine Leitung einer Pumpe aufgesteckt oder aufgeschraubt werden kann. In dem Kanalstumpf ist ein Rückschlagventil vorgesehen, dass schliesst, sobald die Unterdruckeinrichtung abgenommen oder abgeschaltet wird. Ein Unterdruck wird in den Volumina der einzelnen Kammern des Gehäuses der Öffnungsvorrichtung und des Mischsystems erzeugt, je nachdem wie die Stellung der Drehtrommel in der zweiten Gehäusekammer gewählt wird. Über die Ventilfunktion der Drehtrommel kann das Anlegen des Unterdrucks gesteuert werden.

In Figur 7a ist die Öffnungsvorrichtung in einer Stellung gezeigt, in der die Drehtrommel 8 den Auslass 13 abdeckt. Das Ventil ist geschlossen, was auch durch die Querstellung des Griffs 9 angezeigt wird (siehe Figur 7b). Der Anschluss 21 liegt dem Langloch 15 der Drehtrommel 8 gegenüber. Wird nun über den Anschluss 21 ein Unterdruck in den Kammern des Gehäuses der Öffnungsvorrichtung erzeugt, wird in dem Mischbehälter eines angeschlossenen Mischsystems oder auch in weiteren über den Mischbehälter angeschlossenen Öffnungsvorrichtungen kein Unterdruck aufgebaut.

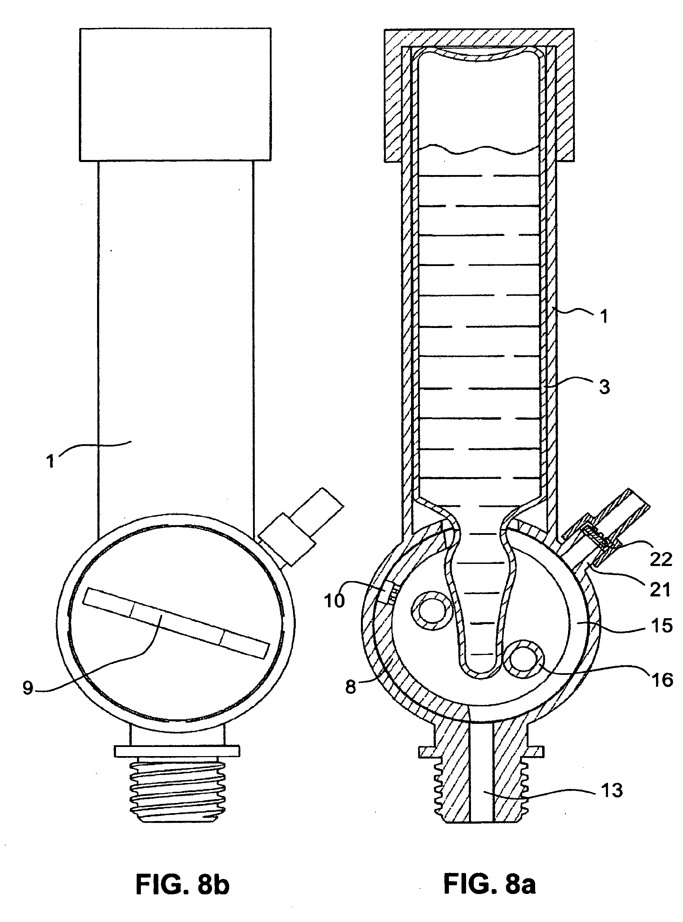

In Figur 8a ist die Drehtrommel 8 in der zweiten Gehäusekammer 2 in einer geöffneten Position gezeigt, in der das Langloch 15 gegenüber dem Auslass 13 zu liegen kommt und somit die Volumina des Gehäuses der Öffnungsvorrichtung und des Mischbehälters eines Mischsystems mit einander verbunden sind. Das Ventil ist somit geöffnet, wie auch an der ersten Schrägstellung des Griffs 9 in Figur 8b zu erkennen ist. Die beiden Stifte 16 sind in dieser Ausführungsform so weit von einander entfernt neben dem Kopfteil 6 der Ampulle angeordnet, dass sie ausreichend Spiel haben, um bei der geringen Drehung der Drehtrommel von der Ausgangsposition in die geöffnete Position den Kopfteil 6 noch nicht abzutrennen. Wird nun ein Unterdruck über den Anschluss 21 an die zweite Gehäusekammer 2 angelegt, wird über den Durchgang durch das Langloch 15 und den Auslass 13, d. h. die geöffnete Ventilfunktion der Drehtrommel, im gesamten System ein Unterdruck erzeugt.

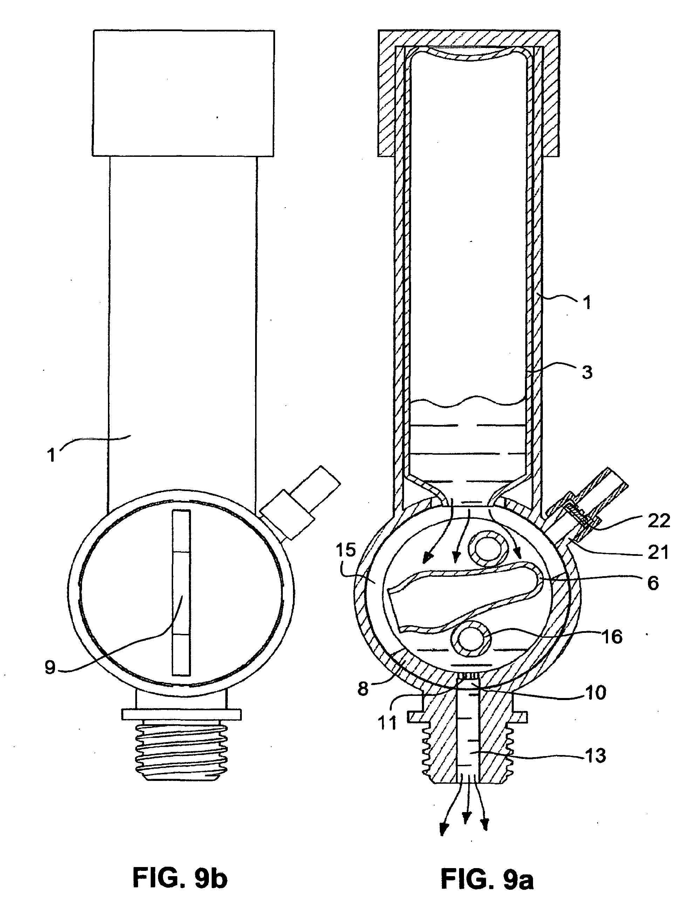

In Figur 9a befindet sich die Drehtrommel 8 in einer Trennposition, in der zum Einen das Kopfteil 6 von der Ampulle 3 mittels der Stifte 16 abgetrennt wurde und zum Anderen der Filter 11 gegenüber dem Auslass 13 zu liegen kommt. Sobald das Kopfteil 6 abgetrennt wird, wird der Inhalt der Ampulle aus dieser herausgesaugt, bzw. herausgedrückt, da im Inneren der Ampulle ein höherer Druck herrscht als in den Volumina des Mischsystems. Das Fluid wird daher zuverlässig vollständig aus der Ampulle ausgeschüttet. In dieser Position ist das Ventil ebenfalls geöffnet, wie an der Längsstellung des Griffs 9 in Figur 9b abzulesen ist, der Auslass 13 wird jedoch durch den Filter 11 abgedeckt.

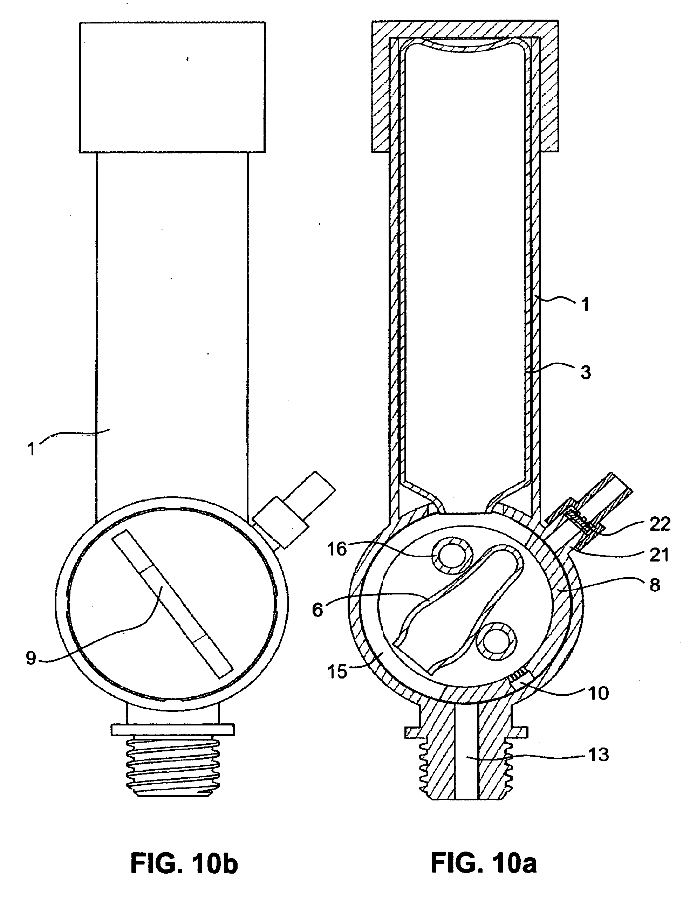

In Figur 10a ist die Drehtrommel 8 wieder in eine Verschlussposition gedreht gezeigt, in der die Umfangswand der Drehtrommel den Ausgang 13 verdeckt und abschliesst. Dies ist an der zweiten Schrägstellung des Griffs 9 zu erkennen, die der ersten Schrägstellung entgegengesetzt ist (siehe Figur 10b). Der Unterdruck ist im System immer noch aufrecht erhalten, da sowohl das Rückschlagventil geschlossen ist, als auch die Verbindung zwischen dem Mischbehälter und der Öffnungsvorrichtung weiterhin besteht. Die Umfangswand der Drehtrommel verschliesst in dieser Stellung den Unterdruckanschluss. In diesem Zustand kann das Gemisch blasenfrei gemischt werden. Anschliessend kann die Öffnungsvorrichtung abgenommen werden oder auch nur ein Kanüle zur Abgabe an den Mischbehälter angesetzt werden.

Auf der Aussenseite des Gehäuse der Öffnungsvorrichtung kann eine Beschriftung entsprechend der einzelnen Stellungen des Griffs 9 gemäss den Figuren 7b bis 10b angebracht werden, mit deren Hilfe die Position der Drehtrommel und damit der Zustand des Mischsystems in einfacher Weise abgelesen werden können.

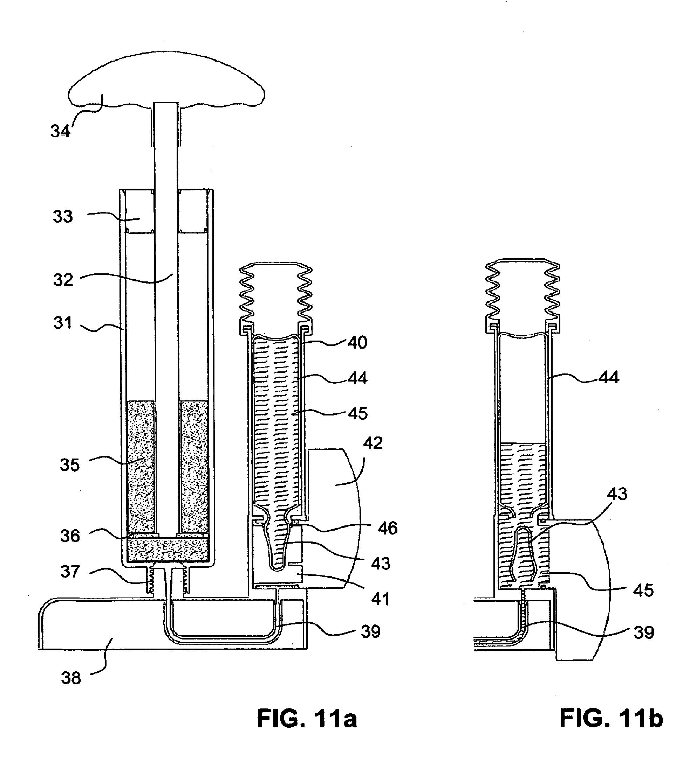

In den Figuren 11a und 11b ist ein System zum Mischen zweier Komponenten mit einer dritte Ausführungsform einer erfindungsgemässen Öffnungsvorrichtung dargestellt. Das System weist einerseits eine Mischvorrichtung, andererseits die damit verbundene Öffnungsvorrichtung auf.

Die Mischvorrichtung weist eine Mischkammer 31 mit einer darin aufgenommenen ersten, z.B. pulverförmigen Komponente 35 auf. Die Mischkammer ist an ihrem oberen Ende durch einen Stopfen 33 verschlossen, in dem eine Kolbenstange 32 geführt ist. Am oberen Ende der Kolbenstange ist ein Handgriff 34 angebracht. Ein Mischelement 36, das z.B. die Form einer Lochplatte annehmen kann, ist im Inneren der Mischkammer 31 am anderen Ende der Kolbenstange 32 befestigt.

Über einen Anschluss 37, z.B. eine Bajonett- oder Schraubverbindung, ist die Mischvorrichtung lösbar mit einem Basisteil 38 verbunden, in dem eine Leitung 39 verläuft. Die Leitung 39 bildet eine Fluidverbindung zwischen der Mischvorrichtung und der Öffnungsvorrichtung. An einem oder beiden Enden der Leitung 39 kann jeweils ein Filter vorgesehen sein, um die erste Komponente 35 in der Mischkammer zurückzuhalten bzw. Verunreinigungen oder Splitter, die beispielsweise durch das Öffnen der Ampulle entstehen können, in der Öffnungsvorrichtung zurückzuhalten. An der Rückseite der Mischkammer 31 weist die Mischkammer im oberen Teil einen Anschlussstutzen (nicht gezeigt) für eine Unterdruckeinrichtung auf, die in der Mischkammer einen Unterdruck erzeugen kann.

Auf dem Basisteil 38 ist die Öffnungsvorrichtung angebracht. Diese umfasst ein Gehäuse 40, in das von oben her eine Ampulle 44 mit ihrem Kopf 43 voraus eingeschoben ist. In der Ampulle 44 ist eine zweite, flüssige Komponente 45 vorhanden. Nach oben hin ist das Gehäuse 40 durch einen faltenbalgartigen Verschluss verschlossen.

Der Ampullenkopf 43 kommt in einer Drehtrommel 41 zu liegen, die in einem unteren, trommelartigen Gehäusebereich angeordnet und hier nur schematisch dargestellt ist. Über einen O-Ring ist die Drehtrommel 41 gegenüber dem Gehäuse 40 abgedichtet. Die Drehtrommel 41 ist um eine quer zur Ampullenlängsachse angeordnete Drehachse im Gehäuse 40 drehbar. Sie ist mit einem Griff 42 in Form einer aufgestellten Platte versehen, die seitlich in der Verlängerung der Drehachse der Drehtrommel 41 von dieser abragt. Der Griff 42 ist dabei bezüglich der Drehachse asymmetrisch ausgestaltet und ragt mit einem Griffende radial über die Umfangswand der Drehtrommel hinaus. In der Ausgangsstellung der Fig. 11a weist der überstehende Bereich des Griffs nach oben, weg vom Basisteil 38, und zeigt so an, dass sich die Drehtrommel in der Ausgangsstellung befindet, in der die Ampulle 44 verschlossen ist.

In Fig. 11b wurde die Drehtrommel um 180° in eine Trennposition gedreht, in der die Drehtrommel den Ampullenkopf 43 vom Körper der Ampulle 44 abgetrennt hat. Der überstehende Bereich des Griffs weist nun nach unten, in Richtung des Basisteils 38, und zeigt an, dass die Ampulle geöffnet wurde. Durch den überstehenden Bereich kann der Griff vom Benutzer sicher ergriffen werden. Der überstehende Bereich ermöglicht beim Drehen eine erhöhte Hebelwirkung, so dass das Öffnen mit verhältnismässig geringen Kräften möglich ist.

Durch das Öffnen der Ampulle 44 fliesst ein Teil der darin aufgenommenen Komponente 45 in die Leitung 39 und in die Mischkammer 31. Zusätzlich wird nun die Unterdruckeinrichtung aktiviert, wodurch in der Mischkammer 31 ein Unterdruck erzeugt wird. Dadurch wird der Rest der zweiten Komponente 45 durch die Leitung 39 in die Mischkammer 31 befördert. Dabei werden sowohl die Ampulle 44 als auch der Ampullenkopf 43, der mit der Öffnung nach unten von der Drehtrommel gehalten wird, entleert. Der Unterdruck ist von aussen dadurch erkennbar, dass sich der Faltenbalg zusammenzieht.

Sobald die zweite Komponente 45 in die Mischkammer 31 umgeleitet wurde, wird die Kolbenstange 32 an einem Griff 34 ergriffen und das Mischelement 36 durch Ein- und Ausschieben und Verdrehen der Kolbenstange 32 in der Mischkammer 31 bewegt, um die Komponenten miteinander zu mischen. Dazu kann die Mischvorrichtung von der Basiseinheit 38 abgenommen werden oder auf dieser verbleiben.

Die Öffnungsvorrichtung ist in dem gezeigten Ausführungsbeispiel fest mit der Basiseinheit 38 verbunden. Alternativ ist es jedoch auch möglich, auch die Öffnungsvorrichtung lösbar mit der Basiseinheit zu verbinden.

### BEZUGSZEICHENLISTE

| | | | |
|---|---|---|---|
| 1 | Erste Gehäusekammer | 13 | Auslass |
| 2 | Zweite Gehäusekammer | 14 | Anschlussstück |
| 3 | Glasampulle | 15 | Langloch |
| 4 | Verschlusskappe | 16 | Stift |
| 5 | Öffnung | 17 | Führungskante |
| 6 | Kopfteil / Glasampulle | 18 | Mischbehälter |
| 7 | Schulter | 19 | Anschlussstück |
| 8 | Drehtrommel | 20 | Mischpaddel |
| 9 | Griff | 21 | Unterdruckanschluss |
| 10 | Durchgang | 22 | Rückschlagventil |
| 11 | Filter | | |
| 12 | Dichtring | | |

## Patentansprüche

1. Öffnungsvorrichtung zum Öffnen eines verschlossenen Fluidbehälters, umfassend:
ein Gehäuse mit einer ersten Kammer (1) zur Aufnahme des Fluidbehälters (3) und einer mit der ersten Kammer verbundenen zweiten Kammer (2) mit einem Auslass (13), wobei die zweite Kammer (2) dazu ausgebildet ist, einen abtrennbaren Teil (6) des Fluidbehälters aufzunehmen, und
eine um eine Drehachse drehbar in der zweiten Kammer (2) geführte Drehtrommel (8) mit wenigstens einem Trennelement (16), das in einer Ausgangsposition versetzt zum abtrennbaren Behälterteil (6) angeordnet ist und durch eine Drehung der Drehtrommel (8) um die Drehachse in eine Trennposition bringbar ist, in der es den abtrennbaren Behälterteil (6) vom Fluidbehälter (3) trennt,
wobei die erste und die zweite Kammer eine Verbindungsachse definieren und die Drehachse der Drehtrommel (8) gewinkelt zu dieser Verbindungsachse verläuft,
wobei die zweite Kammer (2) trommelartig ausgestaltet ist und eine sich in einer Umfangsrichtung um die Drehachse der Drehtrommel (8) erstreckende Mantelwand aufweist,
**dadurch gekennzeichnet, dass** der Auslass (13) in der Mantelwand der zweiten Kammer (2) ausgebildet ist.

2. Öffnungsvorrichtung nach Anspruch 1, wobei der Auslass (13) der zweiten Kammer (2) entlang der Verbindungsachse und gegenüber der ersten Kammer (1) angeordnet ist.

3. Öffnungsvorrichtung nach Anspruch 1 oder 2, wobei der Auslass (13) der zweiten Kammer in ein Anschlussstück (14) zum lösbaren Anschluss an weitere Einrichtungen mündet.

4. Öffnungsvorrichtung nach einem der vorhergehenden Ansprüche, wobei die Drehtrommel (8) eine Umfangswand mit wenigstens einem Durchgang (10) aufweist, der in der Ausgangsposition der Mantelwand der zweiten Kammer (2) gegenüberliegt, so dass die Umfangswand der Drehtrommel in der Ausgangsposition den Auslass (13) verschliesst, und der in einer verdrehten Position den Auslass (13) freigibt, wodurch eine Ventilfunktion erzielt wird.

5. Öffnungsvorrichtung nach Anspruch 4, wobei der Durchgang der Drehtrommel (8) einen Filter (11) aufweist.

6. Öffnungsvorrichtung nach einem der vorhergehenden Ansprüche, wobei zwischen der zweiten Kammer (2) und der Drehtrommel (8) eine Führung (17) vorgesehen ist mit je einem Drehanschlag in einer ersten Drehrichtung, so dass ein Drehen in der Trennposition gestoppt wird, und in eine zweite Drehrichtung, so dass ein Drehen in der Ausgangsposition gestoppt wird.

7. Öffnungsvorrichtung nach einem der vorhergehenden Ansprüche, wobei die Drehachse der Drehtrommel (8) im Wesentlichen senkrecht zur Verbindungsachse zwischen erster und zweiter Kammer verläuft.

8. Öffnungsvorrichtung nach einem der vorhergehenden Ansprüche, wobei die Drehtrommel (8) einen Griff (9) aufweist, der aus der zweiten Kammer (2) hervorsteht und dazu ausgebildet ist, die Drehtrommel (8) in der zweiten Kammer (2) zu drehen.

9. Öffnungsvorrichtung nach Anspruch 8, wobei der Griff (9) eine Griffplatte umfasst, die von der Drehtrommel absteht.

10. Öffnungsvorrichtung nach Anspruch 8 oder 9, wobei der Griff (9) bezüglich der Drehachse der Drehtrommel (8) asymmetrisch ausgebildet ist.

11. Öffnungsvorrichtung nach einem der vorhergehenden Ansprüche, wobei das Gehäuse einen Anschluss (21) zur Erzeugung eines Unterdrucks in dem Gehäuse aufweist.

12. System zum Mischen wenigstens zweier Komponenten, umfassend:
wenigstens eine Öffnungsvorrichtung nach einem der Ansprüche 1 bis 11 und
einen Mischbehälter (18) mit wenigstens einem Anschlussstück (19), an dem die wenigstens eine Öffnungsvorrichtung unter Herstellung einer Fluidverbindung abnehmbar angeschlossen ist, und einer Mischeinrichtung zum Mischen von Komponenten im Mischbehälter.

13. System nach Anspruch 12, wobei am Mischbehälter ein Anschluss (21) zur Erzeugung eines Unterdrucks innerhalb des Systems vorhanden ist.

14. System nach Anspruch 12 oder 13, wobei an das Anschlussstück des Mischbehälters eine Abgabedüse oder -kanüle anschliessbar ist.

## Claims

1. An opening device for opening a closed fluid container, comprising:
a housing with a first chamber (1) for receiving the fluid container (3) and with a second chamber (2) connected to the first chamber and having an outlet (13), the second chamber (2) being adapted to receive a separable part (6) of the fluid container, and
a rotary drum (8) guided rotatably in the second chamber (2) about a rotation axis and having at least one separating element (16) which, in a starting position, is offset in relation to the separable container part (6) and, by a rotation of the rotary drum (8) about the rotation axis, is adapted to be brought to a separating position, in which it separates the separable container part (6) from the fluid container (3),
the first and second chambers defining a connection axis, and the rotation axis of the rotary drum (8) extending at an angle to this connection axis,
the second chamber (2) being shaped like a drum and having a jacket wall that extends in a circumferential direction about the rotation axis of the rotary drum (8),
**characterized in that** the outlet (13) is formed in the jacket wall of the second chamber (2).

2. The opening device as claimed in claim 1, wherein the outlet (13) of the second chamber (2) is arranged along the connection axis and opposite the first chamber (1).

3. The opening device as claimed in claim 1 or 2, wherein the outlet (13) of the second chamber opens into a connecting piece (14) for releasable attachment to further devices.

4. The opening device as claimed in one of the preceding claims, wherein the rotary drum (8) has a circumferential wall with at least one passage (10) which, in the starting position, lies opposite the jacket wall of the second chamber (2), such that the circumferential wall of the rotary drum closes the outlet (13) in the starting position, and which frees the outlet (13) in a rotated position, as a result of which a valve function is obtained.

5. The opening device as claimed in claim 4, wherein the passage in the rotary drum (8) has a filter (11).

6. The opening device as claimed in one of the preceding claims, wherein a guide (17) is provided between the second chamber (2) and the rotary drum (8), with a rotation limit stop in a first direction of rotation, such that a rotation is stopped in the separating position, and with a rotation limit stop in a second direction of rotation, such that a rotation is stopped in the starting position.

7. The opening device as claimed in one of the preceding claims, wherein the rotation axis of the rotary drum (8) is substantially perpendicular to the connection axis between the first and second chambers.

8. The opening device as claimed in one of the preceding claims, wherein the rotary drum (8) has a handle (9), which protrudes from the second chamber (2) and is designed for rotating the rotary drum (8) in the second chamber (2).

9. The opening device as claimed in claim 8, wherein the handle (9) comprises a grip plate extending from the rotary drum.

10. The opening device as claimed in claim 8 or 9, wherein the handle (9) is configured asymmetrically with respect to the rotation axis of the rotary drum (8).

11. The opening device as claimed in one of the preceding claims, wherein the housing has a connector (21) for generating an underpressure in the housing.

12. A system for mixing at least two components, comprising:
at least one opening device as claimed in one of claims 1 through 11, and
a mixing container (18) with at least one connecting piece (19) to which the at least one opening device is removably connected in order to establish a fluid connection, and with a mixing device for mixing components in the mixing container.

13. The system as claimed in claim 12, wherein a connector (21) for generating an underpressure in the system is present on the mixing container.

14. The system as claimed in claim 12 or 13, wherein the connecting piece of the mixing container is adapted for attachment of a dispensing nozzle or dispensing needle.

## Revendications

1. Dispositif d'ouverture pour ouvrir un réservoir à fluide fermé, comprenant :
un boitier avec un premier compartiment (1) pour recevoir le réservoir à fluide (3) et un deuxième compartiment (2) relié au premier compartiment, avec une sortie (13), le deuxième compartiment (2) étant conçu pour recevoir une partie (6) séparable du réservoir à fluide et
un tambour rotatif (8) guidé en étant rotatif dans le deuxième compartiment (2), avec au moins un élément de séparation (16), qui dans une position initiale est placé en étant déporté par rapport à la partie (6) séparable et qui par une rotation du tambour rotatif (8) autour de l'axe de rotation peut être amené dans une position de séparation, dans laquelle il sépare la partie (6) séparable du réservoir à fluide et le réservoir à fluide (3),
le premier et le deuxième compartiment définissant un axe de liaison et l'axe de rotation du tambour rotatif (8) s'étendant en étant coudé par rapport à cet axe de liaison,
le deuxième compartiment (2) étant conçu à la manière d'un tambour et comportant une enveloppe s'étendant dans un sens périphérique autour de l'axe de rotation du tambour rotatif (8),
**caractérisé en ce que** la sortie (13) est conçue dans la paroi d'enveloppe du deuxième compartiment (2).

2. Dispositif d'ouverture selon la revendication 1, la sortie (13) du deuxième compartiment (2) étant disposée le long de l'axe de rotation et à l'opposé du premier compartiment (1).

3. Dispositif d'ouverture selon la revendication 1 ou la revendication 2, la sortie (13) du deuxième compartiment débouchant dans une pièce de raccordement (14) sur le raccord amovible sur une autre installation.

4. Dispositif d'ouverture selon l'une quelconque des revendications précédentes, le tambour rotatif (8) comportant une paroi périphérique avec au moins un passage (10), qui dans la position initiale de la paroi d'enveloppe est opposée au deuxième compartiment (2), de sorte que la paroi périphérique du tambour rotatif ferme la sortie (13) dans la position initiale et qui dans une position tournée libère la sortie (13), suite à quoi une fonction de soupape est obtenue.

5. Dispositif d'ouverture selon la revendication 4, le passage du tambour rotatif (8) comportant un filtre (11).

6. Dispositif d'ouverture selon l'une quelconque des revendications précédentes, entre le deuxième compartiment (2) et le tambour rotatif (8) étant prévu un guidage (17) avec chaque fois une butée en rotation dans un premier sens de rotation, de sorte qu'une rotation dans la position de séparation soit arrêtée et dans un deuxième sens de rotation, de sorte qu'une rotation dans la position initiale soit arrêtée.

7. Dispositif d'ouverture selon l'une quelconque des revendications précédentes l'axe de rotation du tambour rotatif (8) s'étendant sensiblement à la perpendiculaire de l'axe de rotation entre le premier et le deuxième compartiment.

8. Dispositif d'ouverture selon l'une quelconque des revendications précédentes, le tambour rotatif (8) comportant une poignée (9) qui déborde hors du deuxième compartiment (2) et qui est conçue pour faire tourner le tambour rotatif (8) dans le deuxième compartiment (2).

9. Dispositif d'ouverture selon la revendication 8, la poignée (9) comprenant une plaque de préhension qui déborde du tambour rotatif.

10. Dispositif d'ouverture selon la revendication 8 ou la revendication 9, la poignée (9) étant conçue en étant asymétrique par rapport à l'axe de rotation du tambour rotatif (8).

11. Dispositif d'ouverture selon l'une quelconque des revendications précédentes, le boîtier comportant un raccord (21) pour la création d'une dépression dans le boîtier.

12. Système pour mélanger au moins deux composants, comprenant
au moins un dispositif d'ouverture selon l'une quelconque des revendications 1 à 11 et
un réservoir mélangeur (18) avec au moins une pièce de raccordement (19) sur laquelle l'au moins un dispositif d'ouverture est raccordé de manière amovible en établissant une liaison par fluide, et un système mélangeur pour mélanger des composants dans le réservoir mélangeur.

13. Système selon la revendication 12, sur le réservoir mélangeur se trouvant un raccord (21) pour créer une dépression à l'intérieur du système.

14. Système selon la revendication 12 ou la revendication 13, une buse ou une canule distributrice pouvant se raccorder sur la pièce de raccordement du réservoir mélangeur.
